(19) Europäisches Patentamt / European Patent Office / Office européen des brevets

(11) **EP 2 468 229 A1**

(12) **EUROPEAN PATENT APPLICATION**
published in accordance with Art. 153(4) EPC

(43) Date of publication:
**27.06.2012 Bulletin 2012/26**

(51) Int Cl.:
*A61F 13/15* (2006.01)   *A41B 9/02* (2006.01)

(21) Application number: **10809838.5**

(86) International application number:
**PCT/JP2010/062997**

(22) Date of filing: **02.08.2010**

(87) International publication number:
**WO 2011/021493 (24.02.2011 Gazette 2011/08)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **19.08.2009 JP 2009190291**

(71) Applicant: **Unicharm Corporation Ehime 799-0111 (JP)**

(72) Inventors:
• **ENDO, Hiroko Kanonji-shi Kagawa 769-1602 (JP)**
• **FUJIOKA, Yoshihisa Kanonji-shi Kagawa 769-1602 (JP)**

(74) Representative: **Knights, Rupert Saunders & Dolleymore LLP 9 Rickmansworth Road Watford WD18 0JU (GB)**

(54) **MESH UNDERPANTS AND METHOD OF PUTTING ON SAME**

(57) Provided are mesh underpants and a method of putting on same, whereby soiling due to nighttime leakage is inhibited even if the wearer is unused to wearing an absorbent pad. The provided mesh underpants (1) have a waist opening (4) and a pair of leg openings (5) and can be attached to the outside surface of a roughly-rectangular absorbent pad (10). When worn outside a disposable diaper (20) that is worn so as to cover the outside surface of the absorbent pad (10), the provided mesh underpants can cover roughly the entire length of the disposable diaper (20).

FIG. 1

EP 2 468 229 A1

**Description**

TECHNICAL FIELD

The present invention relates to mesh underpants and a method of putting on the mesh underpants. More specifically, the present invention relates to mesh underpants having a waist opening and a pair of leg openings, and a method of putting on the mesh underpants.

BACKGROUND ART

Conventionally, a pair of shorts has been proposed on which a liquid absorbent pad of a substantially rectangular shape such as a sanitary napkin and an incontinence pad is fixed and that holds the liquid absorbent pad against a wearer's body (for example, see Patent Document 1).

Such shorts are configured with a small extension ratio in a length direction so as to facilitate wearing of the shorts by the wearer.

Patent Document 1: Japanese Unexamined Patent Application Publication No. 2006-144164

DISCLOSURE OF THE INVENTION

Problems to be Solved by the Invention

The abovementioned liquid absorbent pad is used also in nursing care. In nursing care, the liquid absorbent pad and a shorts-like mesh underpants for fixing the liquid absorbent pad are put on to a wearer by caregivers. In such a case, the shorts as proposed in Patent Document 1 that are configured to facilitate wearing by wearers themselves are difficult for the caregivers to put on to wearers.

The mesh underpants are directly put on to the outer side of the liquid absorbent pad. In such a case, a caregiver not familiar with putting on the liquid absorbent pad may let liquid such as urine leak from the liquid absorbent pad, and such leakage may soil the mesh underpants or clothes.

Given this, the present invention is aimed at providing mesh underpants that can prevent soiling due to leakage even when a caregiver is not familiar with putting on the liquid absorbent pad, and a method of putting on the mesh underpants.

Means for Solving the Problems

The present invention relates to mesh underpants having a waist opening and a pair of leg openings, the mesh underpants being wearable on an outer face side of a liquid absorbent pad,

the mesh underpants being configured to cover substantially an entire length of a disposable diaper in a state of being worn on an outer side of the disposable diaper worn to cover the outer face side of the liquid absorbent pad.

An extension ratio of the mesh underpants in a length direction is preferably 60% to 230%.

The mesh underpants are composed of a stretchable sheet-shaped member formed by weaving a fiber-like member; an outer face of the liquid absorbent pad is visible in a state in which the mesh underpants are worn on the outer face side of the liquid absorbent pad; and the outer face of the disposable diaper is visible in a state in which the mesh underpants are worn on the outer face side of the disposable diaper.

The present invention also relates to a method of putting on mesh underpants, including steps of: placing a liquid absorbent pad on an inner face side of a disposable diaper; putting the disposable diaper, in which the liquid absorbent pad is placed, on a wearer; and covering substantially an entire length of the disposable diaper by putting mesh underpants, which has a waist opening and a pair of leg openings, on an outer face side of the disposable diaper.

A developed type disposable diaper is preferably used as the disposable diaper.

Effects of the Invention

According to the present invention, mesh underpants that can prevent soiling due to leakage even when a caregiver is not familiar with putting on the liquid absorbent pad, and a method of putting on the mesh underpants can be provided.

BRIEF DESCRIPTION OF THE DRAWINGS

FIG. 1 is a perspective view illustrating stretchable pants as a preferred embodiment of the mesh underpants of the present invention;

FIG. 2 is a plan view of the stretchable pants shown in FIG. 1;

FIG. 3 is a perspective view illustrating a state in which the stretchable pants shown in FIG. 1 are put on to an outer face side of a liquid absorbent pad;

FIG. 4 is a perspective view illustrating a state in which the stretchable pants shown in FIG. 1 are put on to an outer face side of a disposable diaper;

FIG. 5 is a plan view of the liquid absorbent pad; and

FIG. 6 is an exploded perspective view of the disposable diaper in a developed state, on which the liquid absorbent pad is placed.

PREFERRED MODE FOR CARRYING OUT THE INVENTION

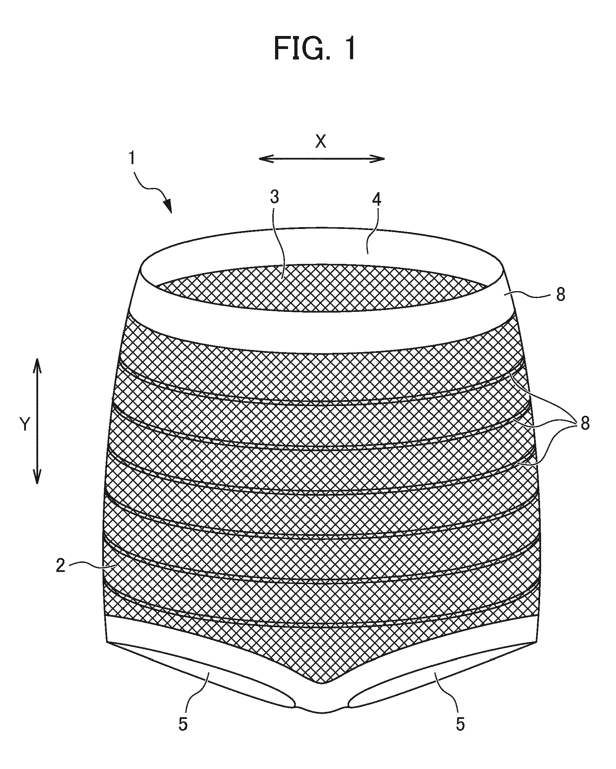

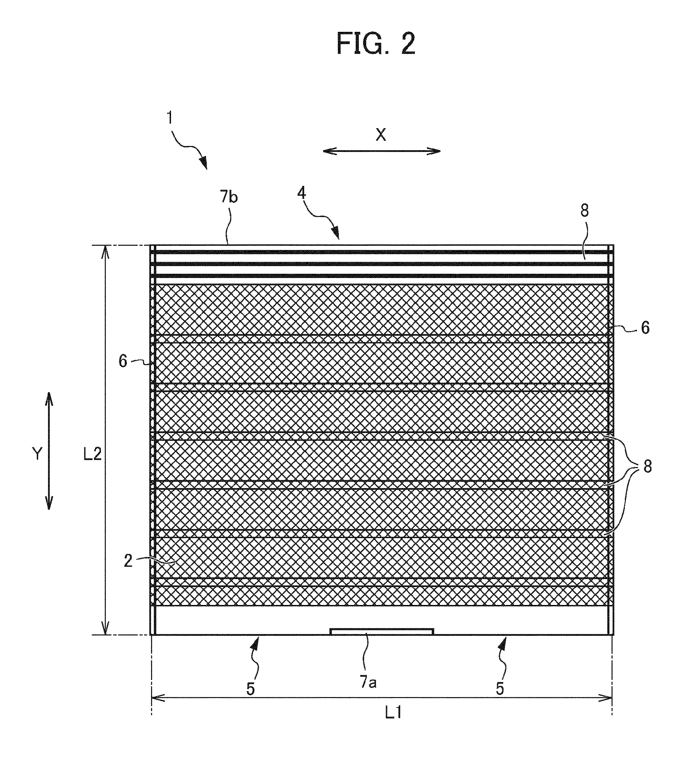

A preferred embodiment of the present invention will be described hereinafter with reference to the drawings. FIG. 1 is a perspective view illustrating stretchable pants as a preferred embodiment of the mesh underpants of the present invention. FIG. 2 is a plan view of the stretchable pants shown in FIG. 1.

As shown in FIGS. 1 and 2, the stretchable pants 1 of the present embodiment have a front side 2 and a back side 3. In addition, the stretchable pants 1 have a waist opening 4 and a pair of leg openings 5.

As shown in FIG. 2, the stretchable pants 1 are formed by, in a state in which two sheets of a wide rectangular sheet-shaped member of substantially the same shape are layered, joining a pair of side edges 6 of the sheet-shaped member along the width direction and joining a substantially central portion of a side edge 7a, which is one of a pair of side edges 7a, 7b of the sheet-shaped member along the longitudinal direction. In other words, non-joined portions in the side edge 7a of the pair of side edges 7a, 7b of the sheet-shaped member along the longitudinal direction forms the pair of leg openings 5, and the side edge 7b forms the waist opening.

The sheet-shaped member composing the stretchable pants 1 are formed by weaving a fiber-like member and has stretchability.

In the vicinity of the waist opening 4 of the stretchable pants 1, an elastic member 8 is provided along the width direction (direction X shown in FIGS. 1 and 2) of the stretchable pants 1. In addition, a plurality of elastic members 8 is arranged between the waist opening 4 and the pair of leg openings 5 of the stretchable pants 1, at predetermined intervals along the width direction of the stretchable pants 1.

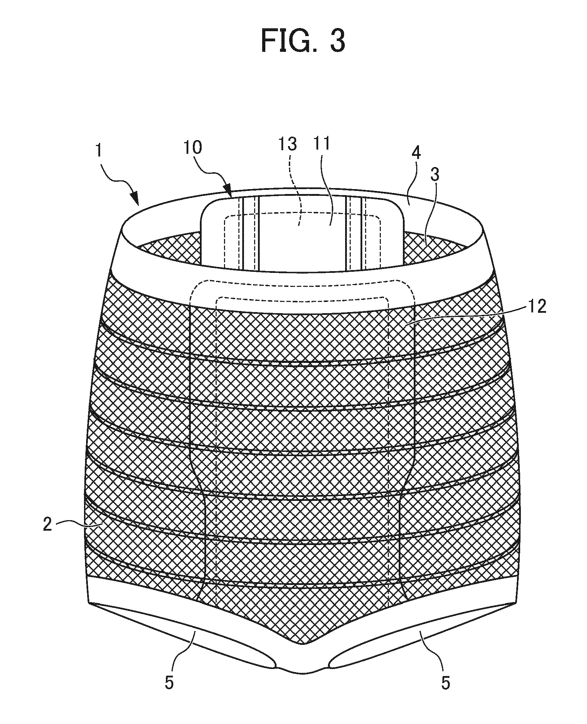

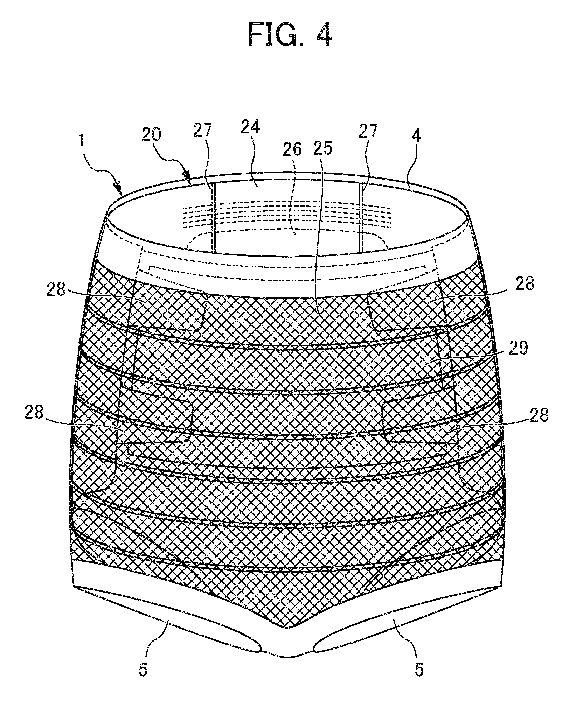

As shown in FIG. 3, in a state in which a substantially rectangular liquid absorbent pad 10 such as a urinary pad is put on to a wearer such as an elderly person, the above-described stretchable pants 1 are put on to the outer face side of the liquid absorbent pad 10. In addition, as shown in FIG. 4, in a state in which the disposable diaper 20 is put on to cover the outer face side of the liquid absorbent pad 10, the stretchable pants 1 are put on to the outer face side of the disposable diaper 20.

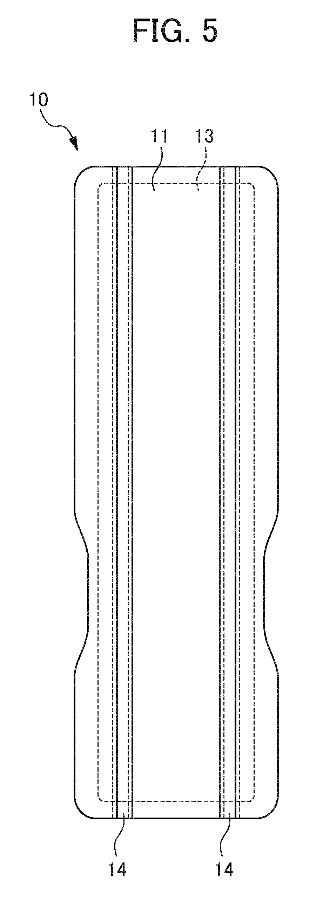

As shown in FIG. 5, the liquid absorbent pad 10 has an elongated rectangular shape in planar view. The liquid absorbent pad 10 is composed of: a liquid permeable top sheet 11 disposed on a skin-contacting side; a liquid impermeable or poorly-permeable back surface sheet 12 (see FIG .3) disposed on a skin-noncontacting side; and a liquid absorptive absorbent core 13 disposed between the top sheet 11 and the back surface sheet 12. In addition, in the vicinities of both side portions of the top sheet 11, a pair of leak-proof walls 14 uprising toward a wearer's skin side is provided.

A length of the liquid absorbent pad 10 in the longitudinal direction is not particularly limited; however, from a viewpoint of appropriately covering the wearer's excretory part, the length is preferably from 120 mm to 960 mm and more preferably from 380 mm to 850 mm. A length of the liquid absorbent pad 10 in the width direction (inside leg width) is preferably from 40 mm to 360 mm and more preferably from 160 mm to 250 mm.

It should be noted that the liquid absorbent pad in the present invention is only required to have a shape and size to substantially cover the excretory part and not limited to the elongated rectangular shape described above. For example, the liquid absorbent pad can be in a solid shape that covers the excretory part. In this case, substantially an entire length of the liquid absorbent pad is a length from an abdomen side to a back side during use.

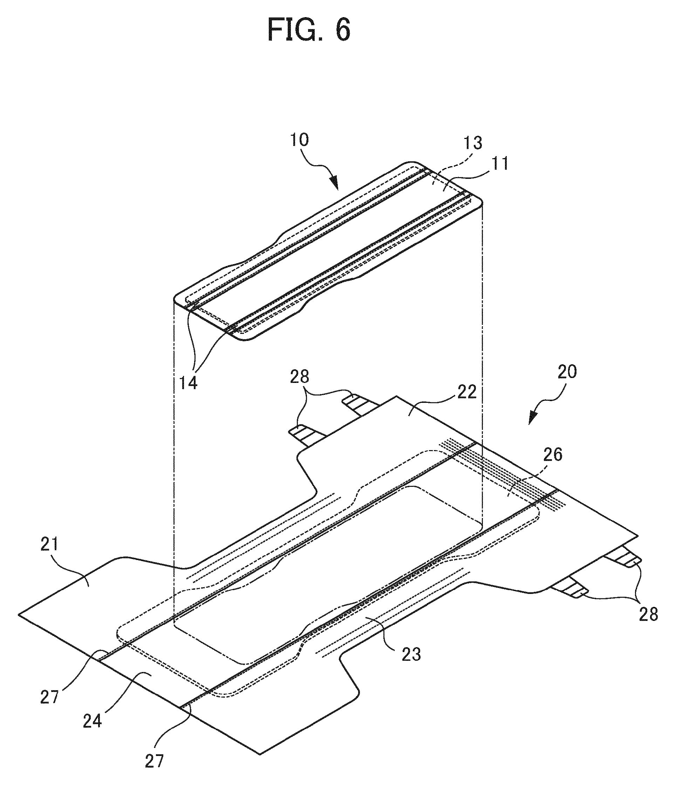

The disposable diaper 20 is a developed type disposable diaper as shown in FIG. 6, composed of: an abdomen side portion 21 disposed on an abdomen side of the wearer during use; a back side portion 22 disposed on a back side of the wearer; an inside leg portion 23 disposed between the abdomen side portion 21 and the back side portion 22. In the disposable diaper 20, both side edges of the inside leg portion 23 are curved inward, forming an hourglass shape that is narrow in a center portion in the longitudinal direction.

The disposable diaper 20 is configured similarly to the liquid absorbent pad 10 and composed of: a liquid permeable top sheet 24 disposed on a skin-contacting side; a liquid impermeable or poorly-permeable back surface sheet 25 (see FIG. 4) disposed on a skin-noncontacting side; and a liquid absorptive absorbent core 26 disposed between the top sheet 24 and the back surface sheet 25, as shown in FIG. 6. In addition, in the vicinities of both side portions of the top sheet 24, a pair of leak-proof walls 27 uprising toward a wearer's skin side is provided.

In addition, in the disposable diaper 20, fastening tapes 28 are provided respectively in both side edges of the back side portion 22 and a landing tape 29 (see FIG. 4) onto which the fastening tapes 28 can be fixed is provided on an outer surface (skin noncontacting surface) of the abdomen side portion.

A length of the disposable diaper 20 in the longitudinal direction is not particularly limited; however, from a viewpoint of appropriately covering the wearer's excretory part, the length is preferably from 300 mm to 1000 mm and more preferably from 790 mm to 960 mm. In addition, a length in the width direction of the crotch portion of the disposable diaper 20 is preferably greater than a length in the width direction of the liquid absorbent pad 10, more specifically from 45 mm to 360 mm.

The stretchable pants 1 of the present embodiment are configured with an extension ratio in the length direction (direction Y shown in FIGS. 1 and 2), which is orthogonal to the width direction X, of 60% to 230%, and preferably 80% to 220 %. The stretchable pants 1 are thus configured to cover substantially an entire length in the longitudinal direction of the liquid absorbent pad 10 in a state of being put on to the outer face side of the liquid absorbent pad 10, and to cover substantially an entire length of the disposable diaper 20 in a state of being put on to the outer face side of the disposable diaper 20.

If the extension ratio of the stretchable pants 1 in the length direction Y is less than 60%, the stretchable pants 1 are difficult to put on to the outer face side of the disposable diaper 20. In addition, the stretchable pants 1 may not be able to cover substantially entire length of the disposable diaper when being put on to the outer face side of the disposable diaper.

On the other hand, if the extension ratio of the stretchable pants 1 in the length direction Y is more than 230%, in a case of putting on the stretchable pants 1 directly on the outer face of the liquid absorbent pad 10, fixing effect for the liquid absorbent pad 10 is insufficient, leading to leakage of liquid.

The extension ratio of the stretchable pants 1 in the width direction X is preferably 40% to 170%, and more preferably 60% to 150%.

The extension ratio of the stretchable pants 1 can be obtained by the following equation:

```
Extension ratio (%) = (Length in maximum extended state -

Length in natural state) / Length in natural state × 100
```

The length LI in the width direction X of the stretchable pants 1 in a natural state is preferably 200 mm to 400 mm and more preferably 230 mm to 350 mm. The length L2 in the length direction Y of the stretchable pants 1 in a natural state is preferably 120 mm to 250 mm and more preferably 140 mm to 230 mm.

A method of putting on the stretchable pants 1 of the present embodiment will be described hereinafter.

As described above, in a state where the liquid absorbent pad 10 is attached to the wearer, the stretchable pants 1 of the present embodiment are put on to the outer face side of the liquid absorbent pad 10 and, even in a state where the disposable diaper 20 is put on in order to cover the outer face side of the liquid permeable pad 10 thus attached, the stretchable pants 1 are put on to the outer face side of the disposable diaper.

First, a method of putting on the stretchable pants 1 directly to the outer face side of the liquid absorbent pad 10 will be described.

In this case, a person (for example, a caregiver) who puts on the liquid absorbent pad 10 and the stretchable pants 1 to the wearer first puts the liquid absorbent pad 10 to cover a crotch part of the wearer. The caregiver then puts the wearer's legs through the pair of leg openings 5 from the waist opening 4 side of the stretchable pants 1. Thereafter, the caregiver pulls up the stretchable pants 1, through the pair of leg openings 5 of which the wearer's legs are put, to cover substantially an entire length of the liquid absorbent pad 10 in the length direction. The liquid absorbent pad 10 is thus covered by the stretchable pants 1 and supported at a position of covering the crotch part of the wearer (see FIG. 3).

In this state, the liquid absorbent pad 10 is visible through the woven fiber-like member composing the sheet-shaped member.

Next, a method of putting on the stretchable pants 1 on the outer face side of the disposable diaper 20 attached to cover the outer face side of the liquid absorbent pad 10 will be described.

In this case, as shown in FIG. 6, the liquid absorbent pad 10 is first placed on the top sheet 24 side (skin contacting side) of the disposable diaper. The caregiver then places the disposable diaper 20 such that the liquid absorbent pad 10 faces the crotch part of the wearer. Thereafter, the caregiver puts the disposable diaper 20 to the wearer by fixing the fastening tapes 28 provided on the back side portion 22 onto the landing tape 29 provided on the outer face side of the abdomen side portion 21. The caregiver then puts the wearer's legs through the pair of leg openings 5 from the waist opening 4 side of the stretchable pants 1. Thereafter, the caregiver pulls up the stretchable pants 1, through the pair of leg openings 5 of which the wearer's legs are put, to cover substantially an entire length of the disposable diaper 20. The liquid absorbent pad 10 is thus covered by the disposable diaper 20 and the stretchable pants 1 and supported at a position of covering the crotch part of the wearer (see FIG. 4).

Also in this state, the disposable diaper 20 is visible through the woven fiber-like member composing the sheet-shaped member.

The stretchable pants 1 and the method of putting on the stretchable pants 1 according to the present embodiment provide the following effects.

The stretchable pants 1 are configured to cover substantially an entire length of the disposable diaper 20 in a state of being put on to the outer face side of the disposable diaper 20. As the stretchable pants 1 can cover substantially an entire length of the disposable diaper 20 in a state of being put on to the outer face side of the disposable diaper 20, the liquid absorbent pad 10 attached to the wearer is not easily dislocated. Given this, if the caregiver is not familiar with putting on the liquid absorbent pad 10, the caregiver can first place the liquid absorbent pad 10 on the disposable diaper 20, put on the disposable diaper 20 on which the liquid absorbent pad 10 is placed to the wearer, and then put on the stretchable pants 1 on the outer face side of the disposable diaper 20. As a result, even if the caregiver is not familiar with the putting on the liquid absorbent pad 10, the stretchable pants 1 are not easily soiled due to leakage from the liquid absorbent pad 10.

The stretchable pants 1 are configured to cover substantially an entire length of the liquid absorbent pad 10 in a state of being put on to the outer face side of the liquid absorbent pad 10. The stretchable pants 1 can thus cover substantially an entire length of the liquid absorbent pad 10 or the disposable diaper 20 either in a case in which the stretchable pants 1 are directly put on to the outer face side of the liquid absorbent pad 10 or in a case in which the stretchable pants 1 are put on to the outer face side of the disposable diaper 20. As a result, the stretchable pants 1 can be used appropriately either in a case in which the stretchable pants 1 are directly put on to the outer face side of the liquid absorbent pad 10 or in a case in which the stretchable pants 1 are put on to the outer face side of the disposable diaper 20, thereby improving convenience of the stretchable pants 1.

The extension ratio of the stretchable pants 1 in the length direction Y is configured to be 60 % to 230 %. The stretchable pants 1 can thus cover substantially an entire length of the liquid absorbent pad 10 or the disposable diaper 20 appropriately, either in a case in which the stretchable pants 1 are directly put on to the outer face side of the liquid absorbent pad 10 or in a case in which the stretchable pants 1 are put on to the outer face side of the disposable diaper 20. This can improve a dislocation prevention effect for the liquid absorbent pad 10 of the stretchable pants 1.

The stretchable pants 1 are composed of the stretchable sheet-shaped member formed by weaving the fiber-like member. The liquid absorbent pad 10 or the disposable diaper 20 can thus be visible through the woven fiber-like member, either in a case in which the stretchable pants 1 are put on to the outer face side of the liquid absorbent pad 10 or in a case in which the stretchable pants 1 are put on to the outer face side of the disposable diaper 20. For example, in a case in which a notification member, which notifies time to replace the liquid absorbent pad 10 or the disposable diaper 20 by change in color upon detecting a predetermined amount of urine, is provided on the outer face of the liquid absorbent pad 10 or the disposable diaper 20, the change in color of the notification member can be easily recognized.

When the wearer changes his posture, for example by rolling over, the liquid absorbent pad 10 disposed between the disposable diaper 20 and the wearer's body can be easily dislocated. In a case in which the liquid absorbent pad 10 is thus dislocated, liquid may be easily leaked to the disposable diaper 20 and may accelerate change-out rate of the disposable diaper 20.

Given this, after putting on the disposable diaper 20 in which the liquid absorbent pad 10 is placed to the wearer, the stretchable pants 1 are put on to the outer face side of the disposable diaper 20 to cover the substantially entire length of the disposable diaper 20. As a result, even when the wearer changes his posture, for example by rolling over, the liquid absorbent pad 10 is not easily dislocated. This can prevent leakage of liquid to the disposable diaper and can reduce the change-out rate of the disposable diaper 20.

As the disposable diaper 20 to be put on to the outer face side of the liquid absorbent pad 10, a developed type disposable diaper is used. The liquid absorbent pad 10 can thus be placed on an inner face side of the disposable diaper 20. The liquid absorbent pad 10 can thus be appropriately placed on the crotch part of the wearer, thereby preventing leakage of liquid to the disposable diaper 20.

A preferred embodiment of the present invention has been explained above; however, the present invention is not limited thereto and can be modified appropriately.

For example, in the present embodiment, the liquid absorbent pad 10 is composed of the top sheet 11, the back surface sheet 12 and the absorbent core 13; however, the present invention is not limited thereto. The liquid absorbent pad can also be composed of a urine receiver including: a liquid permeable top sheet; a liquid impermeable back surface sheet; a urine detector that detects urine discharged by the wearer; a urine ejection tube that ejects the discharged urine; and an ejection mechanism that ejects the urine via the urine ejection tube upon detection of urine by the urine detector.

EXAMPLES

The present invention will be described in further detail hereinafter with reference to Example and Comparative Example.

With Example 1 and Comparative Example 1, an amount of leakage of liquid from the liquid absorbent pad to the disposable diaper (hereinafter referred to as "inner leakage amount") and occurrence of leakage of liquid from the disposable diaper to the outside (hereinafter referred to as "outward leakage") were assessed when the wearer changes his posture after wearing the liquid absorbent pad, the disposable diaper, and the stretchable pants.

Example 1

Stretchable pants: 255 mm in width and 210 mm in length in natural state; 150% extension ratio in width direction; 140% extension ratio in length direction

Liquid absorbent pad: Trade name "Lifree Ohada Kaiteki Pad" Manufactured by Unicharm Corporation

Disposable diaper: Trade name "Lifree Yokomore Anshin Tape Dome" (developed type disposable diaper) Manufactured by Unicharm Corporation

Measurement of Inner Leakage Amount and Outward Leakage Amount

After putting on the liquid absorbent pad and the disposable diaper to three life-size models (dolls), the stretchable pants were put on to the outer face side of the disposable diaper. Next, the models wearing the liquid absorbent pad, the disposable diaper, and the stretchable pants were subjected to 10 artificial sit-ups by jacking up the model 10 times. After the sit-ups, the models were posed respectively in a dorsal position, a lateral position, and a seated position, and 150 ml of artificial urine was injected to each of the models. After injection of the artificial urine, the inner leakage amount and occurrence of outward leakage were assessed.

Comparative example 1

The inner leakage amount and occurrence of outward leakage were assessed in the same method as in Example 1 except for not using the stretchable pants.

Results of assessment of the inner leakage amount and occurrence of outward leakage in Example 1 and Comparative Example 1 are shown in Table 1.

[Table 1]

|  |  | Inner Leakage Amount (ml) | Outward Leakage |
|---|---|---|---|
| Example 1 | Dorsal Position | 0 | Not Observed |
|  | Lateral Position | 0 | Not Observed |
|  | Seated Position | 0.82 | Not Observed |
| Comparative Example 1 | Dorsal Position | 0 | Not Observed |
|  | Lateral Position | 77.38 | Not Observed |
|  | Seated Position | 9.23 | Not Observed |

As shown in Table 1, slight inner leakage was observed in Example 1 in the seated position; however, no inner leakage was observed in the dorsal position and the lateral position. On the other hand, a large amount of inner leakage was observed in Comparative Example in the lateral position and the seated position.

**Claims**

1. Mesh underpants having a waist opening and a pair of leg openings, the mesh underpants being wearable on an outer face side of a liquid absorbent pad, wherein
   the mesh underpants are configured to cover substantially an entire length of a disposable diaper in a state of being worn on an outer side of the disposable diaper worn to cover the outer face side of the liquid absorbent pad.

2. The mesh underpants according to claim 1, wherein an extension ratio in a length direction is 60% to 230%.

3. The mesh underpants according to claim 1 or 2, wherein: the mesh underpants are composed of a stretchable sheet-shaped member formed by weaving a fiber-like member; an outer face of the liquid absorbent pad is visible in a state in which the mesh underpants are worn on the outer face side of the liquid absorbent pad; and the outer face of the disposable diaper is visible in a state in which the mesh underpants are worn on the outer face side of the disposable diaper.

4. A method of putting on mesh underpants, comprising steps of: placing a liquid absorbent pad on an inner face side of a disposable diaper;
   putting the disposable diaper, in which the liquid absorbent pad is placed, on a wearer; and
   covering substantially an entire length of the disposable diaper by putting mesh underpants, which has a waist opening and a pair of leg openings, on an outer face side of the disposable diaper.

5. The method of putting on mesh underpants according to claim 4, wherein a developed type disposable diaper is used as the disposable diaper.

FIG. 1

# FIG. 2

# FIG. 3

# FIG. 4

## FIG. 5

# FIG. 6

## INTERNATIONAL SEARCH REPORT

International application No.

PCT/JP2010/062997

A. CLASSIFICATION OF SUBJECT MATTER
*A61F13/15*(2006.01)i, *A41B9/02*(2006.01)i

According to International Patent Classification (IPC) or to both national classification and IPC

B. FIELDS SEARCHED

Minimum documentation searched (classification system followed by classification symbols)
A61F13/15, A41B9/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | Microfilm of the specification and drawings annexed to the request of Japanese Utility Model Application No. 009532/1978(Laid-open No. 113633/1979) (Honshu Hairaifu Kabushiki Kaisha), 09 August 1979 (09.08.1979), fig. 3; entire text (Family: none) | 1-5 |
| Y | CD-ROM of the specification and drawings annexed to the request of Japanese Utility Model Application No. 95379/1991(Laid-open No. 37219/1993) (Uni-Charm Corp.), 21 May 1993 (21.05.1993), paragraphs [0002], [0013], [0015]; fig. 1 (Family: none) | 1-5 |

☒ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>07 October, 2010 (07.10.10) | Date of mailing of the international search report<br>19 October, 2010 (19.10.10) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2010/062997

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2008-302009 A (Kao Corp.), 18 December 2008 (18.12.2008), paragraph [0017] (Family: none) | 1-3 |
| Y | JP 8-509400 A (Tytex A/S), 08 October 1996 (08.10.1996), fig. 2; page 7, lines 19 to 27 & US 5671615 A    & EP 696912 A & WO 1994/024978 A1    & CN 1122568 A | 3 |
| A | JP 2008-541867 A (Dow Global Technologies Inc.), 27 November 2008 (27.11.2008), entire text; all drawings & US 2007/0032771 A1    & EP 1883382 A & WO 2006/127781 A2    & KR 10-2008-0031179 A & CN 101257872 A | 1-5 |
| A | Microfilm of the specification and drawings annexed to the request of Japanese Utility Model Application No. 137136/1989(Laid-open No. 75727/1991) (Kazuko SUZUKI), 30 July 1991 (30.07.1991), entire text; all drawings (Family: none) | 1-5 |
| A | Microfilm of the specification and drawings annexed to the request of Japanese Utility Model Application No. 116938/1983(Laid-open No. 25702/1985) (Yugen Kaisha Tsukahara), 21 February 1985 (21.02.1985), entire text; all drawings (Family: none) | 1-5 |

Form PCT/ISA/210 (continuation of second sheet) (July 2009)

**EP 2 468 229 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006144164 A